# EUROPEAN PATENT SPECIFICATION

(11) **EP 1 601 635 B1**
(45) Date of publication and mention of the grant of the patent: **09.01.2008**
(21) Application number: 04717253.1
(22) Date of filing: 04.03.2004
(51) Int. Cl.: C07C 29/145, C07C 213/00, C07C 215/20, C07C 33/20, C07C 67/31

(54) **PROCESS FOR PRODUCING OPTICALLY ACTIVE ALCOHOL IN THE PRESENCE OF RHODIUM, A CHIRAL FERROCENYLDIPHOSPHINE AND AN OPTICALLY ACTIVE DIAMINE**
VERFAHREN ZUR HERSTELLUNG VON OPTISCH AKTIVEM ALKOHOL IN GEGENWART VON RHODIUM, EINEM CHIRALEN FERROCENYLDIPHOSPHIN UND EINEM OPTISCH AKTIVEM DIAMIN
PROCEDE DE PRODUCTION D'UN ALCOOL OPTIQUEMENT ACTIF EN PRESENCE DE RHODIUM, FERROCENYLDIPHOSPHINE CHIRALE ET DIAMINE OPTIQUEMENT ACTIVE

(30) Priority: 07.03.2003 JP 2003061843
(43) Date of publication of application: 07.12.2005
(73) Proprietor: Kawaken Fine Chemicals Co., Ltd., Chuo-ku Tokyo 103-0012 (JP)
(72) Inventor: ITO, Toyofumi, Kawaken Fine Chemicals Co., Ltd., Kawagoe-shi, Saitama 3501151 (JP); AOKI, Tsuyoshi, Kawaken Fine Chemicals Co., Ltd, Kawagoe-shi, Saitama 3501151 (JP)
(74) Representative: Jennings, Tara Romaine
(86) International application number: PCT/JP2004/002776
(87) International publication number: WO 2004/078686

(56) References cited:
- US-A- 5 744 606
- HAYASHI T ET AL: "A chiral (hydroxyalkylferrocenyl)phosphine ligand. Highly stereoselective catalytic asymmetric hydrogenation of prochiral carbonyl compounds" TETRAHEDRON LETTERS, ELSEVIER SCIENCE PUBLISHERS, AMSTERDAM, NL, vol. 48, November 1976 (1976-11), pages 4351-4354, XP002123661 ISSN: 0040-4039 cited in the application
- GADAMASETTI, K. G.: "Process Chemistry in the Pharmaceutical Industry" 1999, MARCEL DEKKER , NEW YORK , XP001181975 P. 195 and Table 1, entry 1
- HAYASHI T ET AL: "ASYMMETRIC SYNTHESIS OF 2-AMINO-1-ARYLETHANOLS BY CATALYTIC ASYMMETRIC HYDROGENATION" TETRAHEDRON, ELSEVIER SCIENCE PUBLISHERS, AMSTERDAM, NL, no. 5, 1979, pages 425-428, XP002028704 ISSN: 0040-4020
- W. TANG AND X. ZHANG: "New Chiral Phosphorosus Ligands for Enantioselective Hydrogenation" CHEMICAL REVIEWS, vol. 103, 2003, pages 3029-3069, XP002284690

## Description

### TECHNICAL FIELD

The present invention relates to a process for producing optically active alcohols involving asymmetric hydrogenation of prochiral carbonyl compounds.

### TECHNICAL BACKGROUND

Optically active alcohols, the compounds that the process of the present invention is intended to produce, are useful as starting materials or intermediates for various pharmaceutical products and agrochemicals.

One way to produce optically active alcohols is to asymmetrically hydrogenate a prochiral carbonyl compound in the presence of an asymmetric metal complex catalyst. Japanese Patent Laid-Open Publication No. Hei 8-225466 describes a technique involving asymmetric hydrogenation of a carbonyl compound in the presence of a transition metal catalyst; a base such as a hydroxide of an alkali metal or an alkaline earth metal; and an optically active nitrogen compound. Japanese Patent Laid-Open Publication No. Hei 11-189600 describes a technique in which a ruthenium complex catalyst in which an optically active phosphine and an optically active diamine are coordinated with a ruthenium is used to catalyze hydrogenation of a carbonyl compound. However, the addition of a base often impedes the progress of the reaction and results in a decrease in the enantiomeric excess of the resulting optically active alcohol depending on the types of the carbonyl compound and the catalyst used. Furthermore, even when the ruthenium complex described in Japanese Patent Laid-Open Publication No. Hei 11-189600, in which an optically active phosphine and an optically active diamine are coordinated with a ruthenium, is used, the enantiomeric excess of the resulting alcohol is often insufficient depending on the type of the carbonyl compound used.

There is also known a technique in which a prochiral carbonyl compound is hydrogenated in the presence of a rhodium complex in which an optically active (hydroxyalkylferrocenyl)phosphine is coordinated with a rhodium (see Tetrahedron Lett., 48, 4351 (1976)). In this technique, the enantiomeric excess of the resulting alcohol is often insufficient depending on the type of the carbonyl compound used.

Optically active 3-quinuclidinol is an optically active alcohol suitable for use as a starting material or as an intermediate for various pharmaceutical products. Japanese Patent Laid-Open Publication No. Hei 9-194480 describes a technique in which a quinuclidinone derivative, which is selected from a group of compounds consisting of 3-quinuclidinone, its adducts with Lewis acids, and their corresponding tertiary or quaternary salts, is hydrogenated in the presence of a rhodium, iridium, or ruthenium complex with a chiral diphosphine as ligand. When 3-quinuclidinone undergoes asymmetric hydrogenation, however, the resulting optically active 3-quinuclidinol tends to have an extremely low enantiomeric excess of 20% or less. Also, while the use of tertiary or quaternary salts of 3-quinuclidinone improves the enantiomeric excess, the process requires relatively complicated steps of converting 3-quinuclidinone into a tertiary or quaternary salt and converting the hydrogenated tertiary or quaternary salt into free 3-quinuclidinol. Thus, none of the above-described techniques offer an industrially satisfactory solution.

Accordingly, it is an objective of the present invention to provide a process for asymmetrically hydrogenating prochiral carbonyl compounds that allows high-yield, industrially favorable production of optically active alcohols at a sufficiently high enantiomeric excess.

### DISCLOSURE OF THE INVENTION

In the course of our studies, the present inventors have discovered that, in producing optically active alcohols through asymmetric hydrogenation of prochiral carbonyl compounds, the proportion of a desired enantiomer in the resulting optically active alcohol can be significantly increased by carrying out the hydrogenation in the absence of a base and in the presence of a rhodium complex or a salt thereof; an optically active diphosphine; and an optically active diamine. The discovery inspired the present inventors to ultimately devise the present invention.

Accordingly, a first aspect of the present invention to achieve the above-described objective is a process for producing an optically active alcohol through asymmetric hydrogenation of a prochiral carbonyl compound, characterized in that the hydrogenation is carried out in the presence of:
a rhodium complex or a salt thereof;
an optically active diphosphine ligand of the following general formula (1): 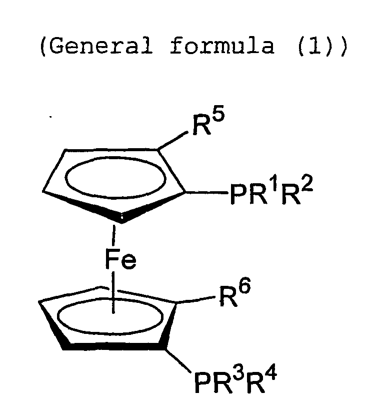 wherein R¹, R², R³, and R⁴ are each independently a substituted or unsubstituted hydrocarbon group containing 1 to 10 carbon atoms; and R⁵ and R⁶ are each independently a hydrogen or a substituted or unsubstituted hydrocarbon group containing 2 to 10 carbon atoms, wherein the substituent is at least one selected from the group consisting of hydroxyl, carbonyl, alkoxyl, amino, and amido; and
an optically active diamine ligand of the following general formula (2): 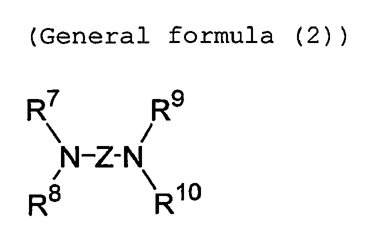 wherein R⁷, R⁸, R⁹, and R¹⁰ are each independently a hydrogen or a substituted or unsubstituted hydrocarbon group; and Z is a hydrocarbon group;
and in the absence of any further base

A second aspect of the invention to achieve the above-described objective is characterized in that, in the first aspect, the optically active diphosphine ligand is one selected from the group consisting of:
a compound of the following general formula (3) 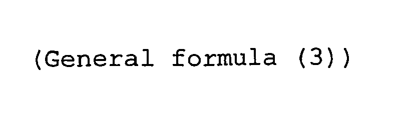 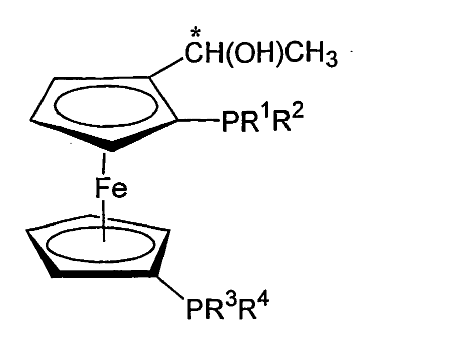 wherein R¹, R², R³, and R⁴ are as described above and * indicates a chiral center; and
a compound of the following general formula (4): 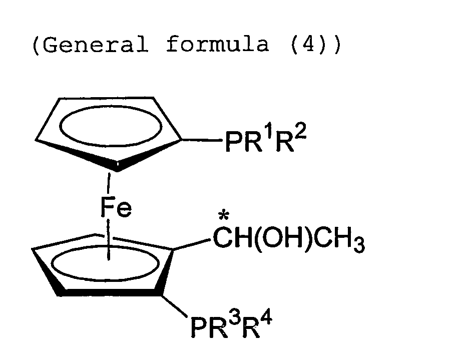 wherein R¹, R², R³, R⁴, and * are as described above.

A third aspect of the invention to achieve the above-described objective is characterized in that, in the first or the second aspect, the prochiral carbonyl compound is 3-quinuclidinone.

### BEST MODE FOR CARRYING OUT THE INVENTION

The present invention will now be described in detail.

Examples of the prochiral carbonyl compounds for use in the present invention include prochiral ketones comprising a carbonyl group bound to a substituted or unsubstituted saturated hydrocarbon group, including 2-butanone, 3-methyl-2-butanone, cyclohexyl methyl ketone, 4-amino-2-butanone, 3-acetyltetrahydrofuran, and N,N-dimethylaminoacetone; prochiral ketones comprising a carbonyl group bound to a substituted or unsubstituted saturated hydrocarbon group and a substituted or unsubstituted unsaturated hydrocarbon group such as a mono- or polycyclic aromatic hydrocarbon group or a mono- or polycyclic heteroaromatic hydrocarbon group, including methyl benzyl ketone, acetophenone, 4'-methylacetophenone, 3'-aminoacetophenone, 2-acetylnaphthalene, 2-acetylfuran, and 3-acetylpyridine; prochiral ketones comprising a carbonyl group bound to a substituted or unsubstituted unsaturated hydrocarbon group such as a mono- or polycyclic aromatic hydrocarbon group or a mono- or polycyclic heteroaromatic hydrocarbon group, including 2-benzoylnaphthalene, and 1-isoquinolinyl phenyl ketone; and substituted or unsubstituted prochiral cyclic ketones, including 2-methylcyclohexanone, β-tetralone, 2,3-dihydro-1H-quinoline-4-one, 3,4-dihydro-2H-1-benzopyran-4-one, and 3-quinuclidinone. Examples of the substituents include hydroxyl, amino, alkoxyl, carbonyl, and amido.

The rhodium complexes or salts thereof for use in the present invention may be any rhodium complex or salt thereof having a structure that allows ligand substitution with the optically active diphosphine and with the optically active diamine to readily take place. Examples include
chloro(cyclooctadiene)rhodium (I) dimer,
bromo(cyclooctadiene)rhodium (I) dimer,
iodo(cyclooctadiene)rhodium (I) dimer,
chloro(cyclooctadiene)(piperidine)rhodium (I),
chloro(cyclooctadiene)(p-toluidine)rhodium (I),
chloro(norbornadiene)rhodium (I) dimer,
acetato(cyclooctadiene)rhodium (I) dimer, L-mandelate(cyclooctadiene)rhodium (I) dimer,
dichlorotetraethylene dirhodium (I), and
bis(norbornadiene)rhodium (I) tetrafluoroborate. The valency of rhodium may vary depending on external conditions, including the types of ligands and solvents, and thus can vary from the valency of atom to the valency of various ions. Specifically, the valency of rhodium varies from 0 to +3. It is preferred that the valency of rhodium is kept to be +1 during the preparation of the hydrogenation catalyst.

The functional groups R¹, R², R³, and R⁴ bound to the phosphorus atom in the optically active diphosphine of the general formula (1) may be each independently an alkyl group, such as methyl, ethyl, n-propyl, and isopropyl; a cycloalkyl group, such as cyclopentyl, and cyclohexyl; an aryl group, such as phenyl, 2-methylphenyl, 4-methylphenyl, 4-methoxyphenyl, and naphthyl; or a benzyl group. The functional groups R⁵ and R⁶ bound to the cyclopentadienyl group in the ferrocene may be each independently hydrogen; an alkyl group, such as ethyl, n-propyl, and isopropyl; an allyl group, such as vinyl; a cycloalkyl group, such as cyclopentyl, and cyclohexyl; an aryl group, such as phenyl, and 2-methylphenyl; or a hydrocarbon group having such substituents as hydroxyl, amino, alkoxyl, carbonyl, and amido, including 2-hydroxyethyl, 1-hydroxyethyl, (1R)-1-hydroxyethyl, (1S)-1-hydroxyethyl, 1-hydroxypropyl, (1R)-1-hydroxypropyl, (1S)-1-hydroxypropyl, 2-(N,N-dimethylamino)ethyl, 1-(N,N-dimethylamino)ethyl, (1R)-1-(N,N-dimethylamino)ethyl, (1S)-1-(N,N-dimethylamino)ethyl, 2-methoxyethyl, 1-methoxyethyl, (1R)-1-methoxyethyl, 2-acetylethyl, 1-acetylethyl, (1R)-1-acetylethyl, (1S)-1-acetylethyl, 1-phenylhydroxymethyl, (1R)-1-phenylhydroxymethyl, (1S)-1-phenylhydroxymethyl, acetyl, and acetamido.

Examples of the optically active diphosphines of the general formula (1) include (R)-1',2-bis(diphenylphosphino)ferrocenylethane, (S)-1',2-bis(diphenylphosphino)ferrocenylethane, (S)-1-[(R)-1',2-bis(diphenylphosphino)ferrocenyl]ethanol (referred to simply as '(S,R)-BPPFOH', hereinafter), (R)-1-[(S)-1',2-bis(diphenylphosphino)ferrocenyl]ethanol (referred to simply as '(R,S)-BPPFOH', hereinafter), (S)-N,N-dimethyl-1-[(R)-1',2-bis(diphenylphosphino)ferrocenyl]ethylamine (referred to simply as '(S,R)-BPPFA)', hereinafter), and (R)-N,N-dimethyl-1-[(S)-1',2-bis(diphenylphosphino)ferrocenyl]ethylamine (referred to simply as '(R,S)-BPPFA)', hereinafter). Of these, preferred are optically active diphosphines of the general formula (1) in which R⁵ or R⁶ is a group having a chiral center, including (S, R) -BPPFOH, (R, S) -BPPFOH, (S, R) -BPPFA, and (R, S) - BPPFA. More preferred are optically active diphosphines of the general formula (3) or (4), including (S,R)-BPPFOH and (R,S)-BPPFOH.

Examples of the optically active diamine of the general formula (2) include (1S,2S)-DPEN:(1S,2S)-1,2-diphenyl-1,2-ethanediamine, (1R,2R)-1,2-diphenyl-1,2-ethanediamine (referred to as '(1R,2R)-DPEN', hereinafter), (1S,2S)-1,2-cyclohexanediamine, (1R,2R)-1,2-cyclohexanediamine, (2S,3S)-2,3-butanediamine, (2R,3R)-2,3-butanediamine, (2S)-1,1-bis(p-methoxyphenyl)-2-isopropyl-1,2-ethanediamine (referred to as '(S)-DAIPEN', hereinafter), (2R)-1,1-bis(p-methoxyphenyl)-2-isopropyl-1,2-ethanediamine(referred to as '(R)-DAIPEN', hereinafter), (2S)-1,1-bisnaphthyl-2-methyl-1,2-ethanediamine, (2R)-1,1-bisnaphthyl-2-methyl-1,2-ethanediamine, (1S,2S)-TsDPEN:N-(p-toluenesulfonyl)-(1S,2S)-1,2-diphenyl-1,2-ethanediamine, N-(p-toluenesulfonyl)-(1R,2R)-1,2-diphenyl-1,2-ethanediamine (referred to as '(1R,2R)-TsDPEN', hereinafter). Of these, optically active DPEN, optically active DAIPEN, and optically active TsDPEN are particularly preferred.

The rhodium complex or a salt thereof is typically used in an amount of 1/50 to 1/10000 molar equivalents, preferably in an amount of 1/500 to 1/2000 molar equivalents, of the carbonyl compound, although it may be used in varying amounts depending on the types of the carbonyl compounds or the catalysts. If the amount of the rhodium complex or a salt thereof is less than 1/10000 molar equivalents, then the reaction will be significantly delayed and some of the carbonyl compound tends to remain unreacted. On the other hand, when used in amounts larger than 1/50 molar equivalents, the rhodium complex or a salt thereof does not produce correspondingly improved effects and makes the process economically disadvantageous.

Preferably, the optically active diphosphine of the general formula (1) is used in an amount of 1.0 to 1.5 molar equivalents of the rhodium. If the compound is used in amounts less than 1.0 molar equivalents, then the enantiomeric excess of the resulting optically active alcohol will be significantly decreased whereas, if the compound is used in amounts exceeding 1.5 molar equivalents, then the reactivity of the catalyst will be significantly reduced, making the process unfavorable.

Preferably, the optically active diamine of the general formula (2) is used in an amount of 0.5 to 2.0 molar equivalents of rhodium. If the compound is used in amounts less than 0.5 molar equivalents, then the optical yield of the resulting optically active alcohol will be significantly reduced, whereas the reactivity of the catalyst will be significantly reduced if the compound is used in amounts exceeding 2.0 molar equivalents.

The solvent for the hydrogenation may be any solvent that can solubilize the carbonyl compound and the catalyst. Specific examples include alcohols, such as methanol, ethanol, 1-propanol, 2-propanol, 1-butanol, and benzyl alcohol; aliphatic and aromatic hydrocarbons, such as pentane, hexane, cyclohexane, methylcyclohexane, benzene, toluene, xylene, and mesitylene; ethers such as diethylether, diisopropylether, tetrahydrofuran, ethyleneglycol dimethylether, and 1,4-dioxane; halogenated hydrocarbons, such as dichloromethane, chloroform, and 1,1,2,2-tetrachloroethane; esters and lactones, such as ethylacetate, and γ-butyrolactone; and carboxamides and lactams, such as N,N-dimethylformamide, N,N-dimethylacetamide, and N-methylpyrrolidone. These solvents may be used either individually or as a mixture of two or more solvents. The use of the solvent may not be necessary in cases where the substrate is provided in liquid state and is capable of solubilizing the catalyst.

The solvent is typically used in an amount of 1/10 to 10,000 times, preferably 1/10 to 100 times the weight of the carbonyl compound. The solvent cannot completely dissolve the carbonyl compound and may lead to a significant decrease in the reactivity when it is used in amounts less than 1/10 the weight of the carbonyl compound. On the other hand, the solvent, when used in amounts more than 10,000 times of the weight of the carbonyl compound, does not bring about correspondingly improved effects and thus makes the process economically unfavorable.

While the hydrogen pressure for use in the present invention may take any value in the range of 0.1 to 20MPa, it is preferably in the range of 0.5 to 10MPa. If the hydrogen pressure is below 0.1MPa, then the progress of the reaction is significantly retarded and some of the carbonyl compound tends to remain unreacted. A hydrogen pressure that is higher than 20MPa, on the other hand, does not bring about correspondingly improved effects and thus is not economically favorable.

While the reaction temperature in the present invention is typically in the range of -50°C to 100°C, it is preferably in the range of 10°C to 40°C. The progress of the reaction is extremely slow and some of the carbonyl compound tends to remain unreacted at temperatures below -50°C, whereas the stability of the complex catalyst and, thus, the enantiomeric excess of the resulting optically active alcohol are significantly reduced at temperatures above 100°C.

Following the reaction, the desired product can be obtained by carrying out isolation/purification using ordinary techniques in synthetic organic chemistry, including solvent extraction, distillation, recrystallization, and chromatography. The structure of the desired product can be determined by ¹H-NMR, polarimetry, high-performance liquid chromatography, gas chromatography, and other known analytical techniques.

### Examples

The present invention will now be described with reference to examples, which are not intended to limit the scope of the invention in any way.

In a glove box with the air inside replaced with nitrogen, a catalyst solution was prepared and was used in a hydrogenation reaction.

The conversion of the carbonyl compound in the hydrogenation reaction was determined by gas chromatography. The enantiomeric excess (%ee) of the resulting optically active alcohol was determined by high-performance liquid chromatography.

### Example 1

4.9mg chloro(cyclooctadiene) rhodium (I) dimer, 13.2g (S,R)-BPPFOH, and 4.5mg (1R,2R)-DPEN were dissolved in 2mL ethanol in a test tube to prepare a catalyst solution. In a separate test tube, 500mg 3-quinuclidinone was dissolved in 8mL ethanol to prepare a substrate solution. The two solutions were then mixed with each other and the mixture was placed in a 200mL stainless steel, pressure-resistant reaction vessel. Hydrogen was then introduced into the vessel to a hydrogen pressure of 3.5MPa and the mixture was stirred for 16 hours at 30°C. Subsequently, the reaction mixture was concentrated under reduced pressure and the solvent was removed. 10mg of the resulting residue was dissolved in 1mL methanol and the solution was subjected to gas chromatography. The results of the analysis showed 100% conversion. The reaction residue was then converted to a butyric acid ester of 3-quinuclidinol by reacting with butyric anhydride and the product was subjected to high-performance liquid chromatography. The results of the analysis revealed that R-3-quinuclidinol was generated at an enantiomeric excess of 68.9%ee.

### Example 2

The process was carried out in the same manner as in Example 1, except that (R,S)-BPPFOH and (1S,2S)-DPEN were used in place of (S,R)-BPPFOH and (1R,2R)-DPEN. S-3-quinuclidinol was obtained at 100% conversion and at an enantiomeric excess of 68.0%ee.

### Comparative Example 1

4.9mg chloro(cyclooctadiene) rhodium (I) dimer, and 13.9mg (S)-1,1'-bisnaphtyl-2,2'-bis(diphenylphosphine) (referred to simply as (S)-BINAP) were dissolved in 2mL THF in a test tube to prepare a catalyst solution. In a separate test tube, 250mg 3-quinuclidinone was dissolved in 8mL ethanol to prepare a substrate solution. The two solutions were then mixed with each other and the mixture was placed in a 200mL stainless steel, pressure-resistant reaction vessel. Hydrogen was then introduced into the vessel to a hydrogen pressure of 3.5MPa and the mixture was stirred for 20 hours at 30°C. Subsequent steps were carried out in the same manner as in Example 1. R-3-quinuclidinol was obtained at 79% conversion and at an enantiomeric excess of 5.6%ee.

### Comparative Example 2

4.9mg chloro(cyclooctadiene)rhodium (I) dimer, 13.9mg (S)-BINAP, and 4.7mg (1R,2R)-DPEN were dissolved in 2mL THF in a test tube to prepare a catalyst solution. In a separate test tube, 250mg 3-quinuclidinone was dissolved in 8mL ethanol to prepare a substrate solution. The two solutions were then mixed with each other and the mixture was placed in a 200mL stainless steel, pressure-resistant reaction vessel. Hydrogen was then introduced into the vessel to a hydrogen pressure of 3.5MPa and the mixture was stirred for 20 hours at 30°C. Subsequent steps were carried out in the same manner as in Example 1. R-3-quinuclidinol was obtained at 21% conversion and at an enantiomeric excess of 5.2%ee.

### Comparative Example 3

2.5mg chloro(cyclooctadiene)rhodium (I) dimer, and 6.6mg (S,R)-BPPFOH were dissolved in 2mL ethanol in a test tube to prepare a catalyst solution. In a separate test tube, 313mg 3-quinuclidinone was dissolved in 8mL ethanol to prepare a substrate solution. The two solutions were then mixed with each other and the mixture was placed in a 200mL stainless steel, pressure-resistant reaction vessel. Hydrogen was then introduced into the vessel to a hydrogen pressure of 3.5MPa and the mixture was stirred for 16 hours at 30°C. Subsequent steps were carried out in the same manner as in Example 1. R-3-quinuclidinol was obtained at 95% conversion and at an enantiomeric excess of 31.6%ee.

### Comparative Example 4

4.9mg chloro(cyclooctadiene)rhodium (I) dimer, 13.2mg (S,R)-BPPFOH, and 4.5mg (1R,2R)-DPEN were dissolved in 2mL ethanol in a test tube to prepare a catalyst solution. In a separate test tube, 313mg 3-quinuclidinone and 112mg potassium t-butoxide was dissolved in 8mL ethanol to prepare a substrate solution. The two solutions were then mixed with each other and the mixture was placed in a 200mL stainless steel, pressure-resistant reaction vessel. Hydrogen was then introduced into the vessel to a hydrogen pressure of 3.5MPa and the mixture was stirred for 16 hours at 30°C. Subsequent steps were carried out in the same manner as in Example 1. R-3-quinuclidinol was obtained at 100% conversion and at an enantiomeric excess of 12.1%ee.

### Comparative Example 5

6.1mg dichloro(p-cymene)ruthenium (II) dimer, 13.2mg (S,R)-BPPFOH, and 4.5mg (1R,2R)-DPEN were dissolved in 2mL ethanol in a test tube to prepare a catalyst solution. In a separate test tube, 500mg 3-quinuclidinone was dissolved in 8mL ethanol to prepare a substrate solution. The two solutions were then mixed with each other and the mixture was placed in a 200mL stainless steel, pressure-resistant reaction vessel. Hydrogen was then introduced into the vessel to a hydrogen pressure of 3.5MPa and the mixture was stirred for 14 hours at 30°C. Subsequent steps were carried out in the same manner as in Example 1. R-3-quinuclidinol was obtained at 8% conversion and at an enantiomeric excess of 22.2%ee.

### Comparative Example 6

6.1mg dichloro(p-cymene)ruthenium (II) dimer, 13.2mg (R,S)-BPPFOH, and 4.5mg (1S,2S)-DPEN were dissolved in 2mL ethanol in a test tube to prepare a catalyst solution. In a separate test tube, 250mg 3-quinuclidinone and 112mg potassium t-butoxide was dissolved in 8mL ethanol to prepare a substrate solution. The two solutions were then mixed with each other and the mixture was placed in a 200mL stainless steel, pressure-resistant reaction vessel. Hydrogen was then introduced into the vessel to a hydrogen pressure of 3.5MPa and the mixture was stirred for 3 days at 30°C. Subsequent steps were carried out in the same manner as in Example 1. S-3-quinuclidinol was obtained at 98% conversion and at an enantiomeric excess of 9.0%ee.

These results are summarized in Table 1 below. The results of Comparative Example 4 clearly indicate that the addition of the base results in a decrease in the enantiomeric excess of the resulting optically active alcohol. Also, it is apparent from the results of Comparative Examples 5 and 6 that, when the hydrogenation is carried out in the presence of the optically active phosphine, the optically active diamine, and the ruthenium complex, the reaction rate is significantly reduced, as is the enantiomeric excess of the resulting optically active alcohol.

### Example 3

The process was carried out in the same manner as in Example 1, except that (R,S)-BPPFOH and 480mg acetophenone were used in place of (S,R)-BPPFOH and 500mg 3-quinuclidinone, respectively. (R)-1-phenylethanol was obtained at 100% conversion and at an enantiomeric excess of 72.1%ee.

### Example 4

The process was carried out in the same manner as in Example 1, except that (R,S)-BPPFOH and 408mg methyl pyruvate were used in place of (S,R)-BPPFOH and 500mg 3-quinuclidinone, respectively. (R)-methyl lactate was obtained at 100% conversion and at an enantiomeric excess of 93.5%ee.

**Table 1**

| | **Catalyst** | **Carbonyl compound** | **S/C** | **Solvent** | **Temper ature (°C)** | **Hydrogen pressure (MPa)** | **Reaction time** | **Conver sion (%)** | **Optical purity (%ee)** | **Config uration** |
|---|---|---|---|---|---|---|---|---|---|---|
| Ex.1 | [Rh(COD)Cl]₂ +(S,R)-BPPFOH+ (1R,2R)-DPEN | 3-quinuclidinone | 200 | ethanol | rt | 3.5 | 16h | 100 | 68.9 | R |
| Ex.2 | [Rh(COD)Cl] ₂ + (R,S)-BPPFOH+ (1S,2S)-DPEN | 3-quinuclidinone | 200 | ethanol | 30 | 3.5 | 16h | 100 | 68.0 | S |
| Comp. Ex.1 | [Rh(COD)Cl] 2 + (S)-BINAP | 3- quinuclidinone | 100 | THF /ethanol | 30 | 3.5 | 20h | 79 | 5.6 | R |
| Comp. Ex.2 | [Rh(COD)Cl] ₂ + (S)- BINAP+ (1 R,2R)-DPEN | 3- quinuclidinone | 100 | THF /ethanol | 30 | 3.5 | 20h | 21 | 5.2 | R |
| Comp. Ex.3 | [Rh(COD)Cl] ₂ + (S,R)-BPPFOH | 3-quinuclidinone | 250 | ethanol | 30 | 3.5 | 16h | 95 | 31.6 | R |
| Comp. Ex.4 | [Rh(COD)Cl] ₂ + (S,R)-BPPFOH+ (1R,2R)-DPEN + t-BuOK | 3-quinuclidinone | 125 | ethanol | 30 | 3.5 | 16h | 100 | 12.1 | R |
| Comp. Ex.5 | [Ru(p-cymene)Cl₂] ₂ + (S,R)-BPPFOH +(1R,2R)-DPEN | 3-quinuclidinone | 200 | ethanol | 30 | 3.5 | 14h | 8 | 22.2 | R |
| Comp. Ex.6 | [Ru(p-cymene)Cl₂] ₂ + (R,S)-BPPFOH +(1828)-DPEN + t-BuOK | 3-quinuclidinone | 125 | ethanol | 30 | 3.5 | 3 days | 98 | 9.0 | S |

| | | | | | | | | | | |
|---|---|---|---|---|---|---|---|---|---|---|
| BINAP: 1,1'-binaphtyl-2,2'-bis(diphenylphosphine) S/C: Molar ratio of carbonyl compound to rhodium complex | | | | | | | | | | |

### INDUSTRIAL APPLICABILITY

As set forth, the present invention makes it possible to produce, in high yield and at a high enantiomeric excess, optically active alcohols from corresponding prochiral carbonyl compounds.

## Claims

1. A process for producing an optically active alcohol through asymmetric hydrogenation of a prochiral carbonyl compound, **characterized in that** the hydrogenation is carried out in the presence of:
a rhodium complex or a salt thereof;
an optically active diphosphine ligand of the following general formula (1): 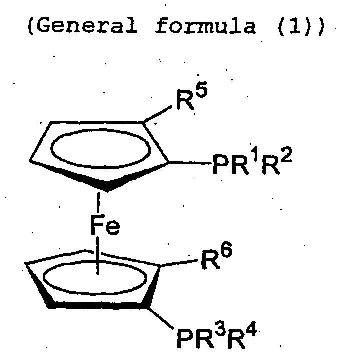 wherein R¹, R², R³, and R⁴ are each independently a substituted or unsubstituted hydrocarbon group containing 1 to 10 carbon atoms; and R⁵ and R⁶ are each independently a hydrogen or a substituted or unsubstituted hydrocarbon group containing 2 to 10 carbon atoms, wherein the substituent is at least one selected from the group consisting hydroxyl, carbonyl, alkoxyl, amino, and amido; and
an optically active diamine ligand of the following general formula (2): 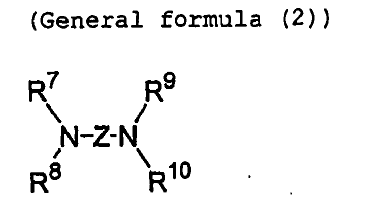 wherein R⁷, R⁸, R⁹, and R¹⁰ are each independently a hydrogen or a substituted or unsubstituted hydrocarbon group; and Z is a hydrocarbon group;
and in the absence of any further base.

2. The process for producing an optically active alcohol according to claim 1, **characterized in that** the optically active diphosphine ligand is one selected from the group consisting of:
a compound of the following general formula (3) 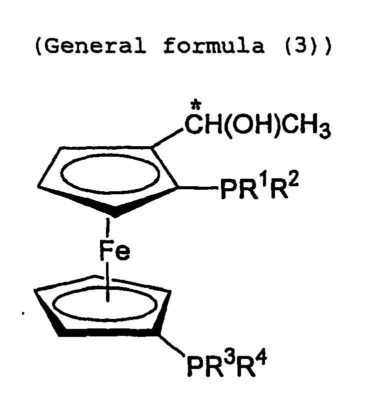 wherein R¹, R², R³, and R⁴ are as described above and * indicates a chiral center; and
a compound of the following general formula (4) : 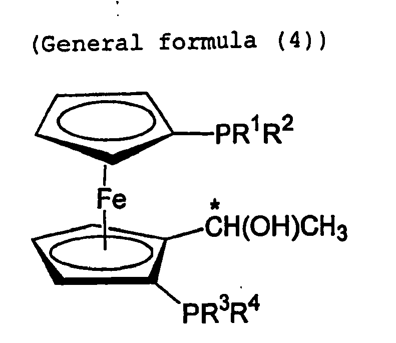 wherein R¹, R², R³, R⁴, and * are as described above.

3. The process for producing an optically active alcohol according to claim 1 or 2, **characterized in that** the prochiral carbonyl compound is 3-quinuclidinone.

## Patentansprüche

1. Verfahren zur Herstellung eines optisch aktiven Alkohols durch asymmetrische Hydrierung einer prochiralen Carbonylverbindung, **dadurch gekennzeichnet, dass** die Hydrierung in Gegenwart von Folgenden durchgeführt wird:
einem Rhodiumkomplex oder einem Salz davon;
einem optisch aktiven Diphosphinliganden der folgenden allgemeinen Formel (1): 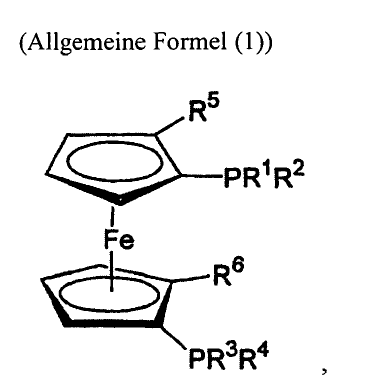 worin R¹, R², R³ und R⁴ jeweils unabhängig eine substituierte oder unsubstituierte Kohlenwasserstoffgruppe sind, die 1 bis 10 Kohlenstoffatome enthält; und R⁵ und R⁶ jeweils unabhängig ein Wasserstoff oder eine substituierte oder unsubstituierte Kohlenwasserstoffgruppe, die 2 bis 10 Kohlenstoffatome enthält, sind, worin der Substituent mindestens eines ist, das aus der Gruppe ausgewählt ist, die aus Hydroxyl, Carbonyl, Alkoxy, Amino und Amido besteht; und
einem optisch aktiven Diaminliganden der folgenden allgemeinen Formel (2): 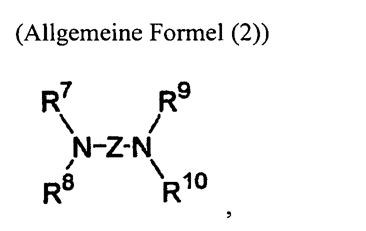 worin R⁷, R⁸, R⁹ und R¹⁰ jeweils unabhängig ein Wasserstoff oder eine substituierte oder unsubstituierte Kohlenwasserstoffgruppe sind; und Z eine Kohlenwasserstoffgruppe ist;
und in Abwesenheit von jeglicher weiterer Base durchgeführt wird.

2. Verfahren zur Herstellung eines optisch aktiven Alkohols nach Anspruch 1, **dadurch gekennzeichnet, dass** der optisch aktive Diphosphinligand eines ist, das aus der Gruppe ausgewählt ist, die aus Folgenden besteht:
einer Verbindung der folgenden allgemeinen Formel (3) 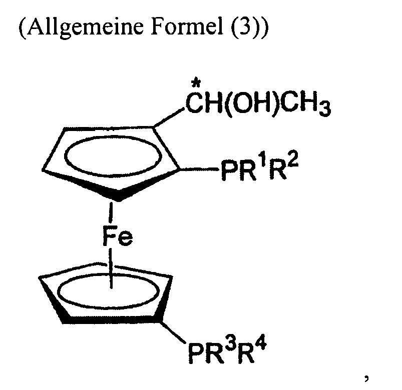 worin R¹, R², R³ und R⁴ wie vorstehend beschrieben sind und * ein chirales Zentrum angibt; und
einer Verbindung der folgenden allgemeinen Formel (4): 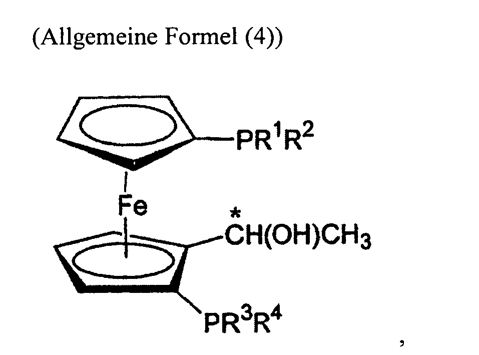 worin R¹, R², R³, R⁴ und * wie vorstehend beschrieben sind.

3. Verfahren zur Herstellung eines optisch aktiven Alkohols nach Anspruch 1 oder 2, **dadurch gekennzeichnet, dass** die prochirale Carbonylverbindung 3-Chinuclidinon ist.

## Revendications

1. Procédé de production d'un alcool optiquement actif par l'intermédiaire d'une hydrogénation asymétrique d'un composé carbonyle prochiral, **caractérisé en ce que** l'hydrogénation est effectuée en présence :
d'un complexe de rhodium ou d'un sel de celui-ci ;
d'un ligand diphosphine optiquement actif de formule générale (1) suivante : 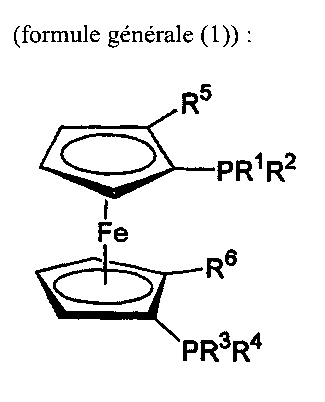 dans laquelle R¹, R², R³ et R⁴ sont chacun indépendamment un groupe hydrocarboné substitué ou non substitué renfermant 1 à 10 atomes de carbone ; et R⁵ et R⁶ sont chacun indépendamment un atome d'hydrogène ou un groupe hydrocarboné substitué ou non substitué renfermant 2 à 10 atomes de carbone, où le substituant est au moins un substituant choisi dans le groupe constitué d'un hydroxyle, d'un carbonyle, d'un alcoxyle, d'un amino et d'un amido ; et
d'un ligand diamine optiquement actif de formule générale (2) suivante : 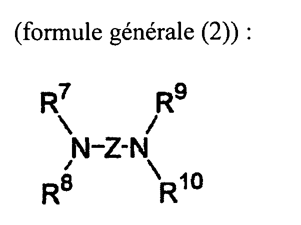 dans laquelle R⁷, R⁸, R⁹ et R¹⁰ sont chacun indépendamment un atome d'hydrogène ou un groupe hydrocarboné substitué ou non substitué ; et Z est un groupe hydrocarboné ; et en l'absence de toute autre base.

2. Procédé de production d'un alcool optiquement actif selon la revendication 1, **caractérisé en ce que** le ligand diphosphine optiquement actif est un ligand choisi dans le groupe constitué :
d'un composé de formule générale (3) suivante : 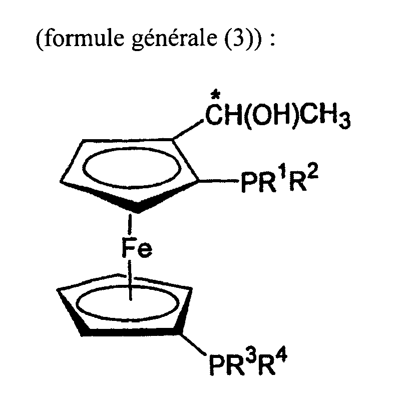 dans laquelle R¹, R², R³ et R⁴ sont tels que décrits ci-dessus et * indique un centre chiral ; et
d'un composé de formule générale (4) suivante : 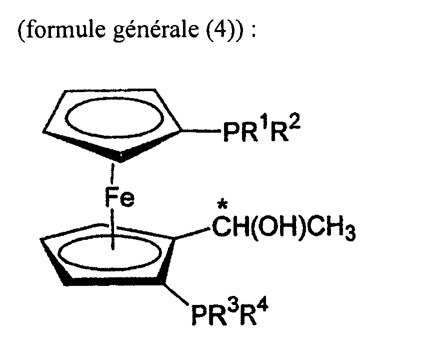 dans laquelle R¹, R², R³, R⁴ et * sont tels que décrits ci-dessus.

3. Procédé de production d'un alcool optiquement actif selon la revendication 1 ou 2, **caractérisé en ce que** le composé carbonyle prochiral est la 3-quinuclidinone.
